(19) Europäisches Patentamt
European Patent Office
Office européen des brevets

(11) EP 4 684 838 A1

(12) EUROPEAN PATENT APPLICATION

(43) Date of publication:
**28.01.2026 Bulletin 2026/05**

(21) Application number: **25189172.7**

(22) Date of filing: **11.07.2025**

(51) International Patent Classification (IPC):
***A61Q 19/00*** (2006.01) ***A61K 8/64*** (2006.01)
***A61K 38/00*** (2006.01) ***A61K 38/04*** (2006.01)

(52) Cooperative Patent Classification (CPC):
**A61Q 19/00; A61K 8/64; A61K 38/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.07.2024 CN 202410993591**

(71) Applicant: **Changchun Ideal Medical Technology Development Co., Ltd**
**Changchun, Jilin 130000 (CN)**

(72) Inventors:
- **ZHU, Lintao**
  **Changchun, Jilin, 130000 (CN)**
- **AFONINA, Irina Vasil'evna**
  **Changchun, Jilin, 130000 (CN)**
- **KOLOBOV, Alexandr Alexandrovich**
  **Changchun, Jilin, 130000 (CN)**
- **GAO, Xiaoyu**
  **Changchun, Jilin, 130000 (CN)**
- **TAN, Liguo**
  **Changchun, Jilin, 130000 (CN)**
- **LU, Qihong**
  **Changchun, Jilin 130000, 130000 (CN)**
- **YIN, Fei**
  **Changchun, Jilin, 130000 (CN)**

(74) Representative: **Curell Suñol S.L.P.**
**Muntaner 240, 4º 2ª**
**08021 Barcelona (ES)**

Remarks:
The complete document including Reference Table(s) and the Sequence Listing(s) can be downloaded from the EPO website

# (54) USE OF POLYPEPTIDE-IN37 AND SKINCARE PRODUCT CONTAINING POLYPEPTIDE-IN37

(57) The present disclosure relates to the field of polypeptide skincare, and in particular, to novel use of a known polypeptide. Specifically disclosed are use of polypeptide-in37 and a skincare product containing polypeptide-in37, namely, use of polypeptide-in37 in any one of the following (1)-(3): (1) preparation of a composition with skin repairing efficacy; (2) preparation of a composition with skin soothing efficacy; and (3) a cream formulation, an emulsions, a gel, an essence, a toner, a shampoo, a shower gel, a facial mask, and a lyophilized product. Polypeptide-in37 is composed of 13 amino acids with a molecular weight of 1,711Da (below 2,000 Da), making it suitable for skin absorption. When applied to the skin as a skin protectant, polypeptide-in37 exhibits safety and excellent functions in skin repairing and skin soothing.

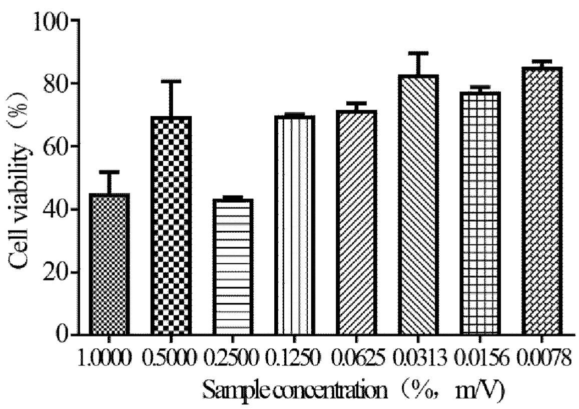


FIG. 1

EP 4 684 838 A1

## Description

### TECHNICAL FIELD

The present disclosure relates to the field of polypeptide-based skincare technology, and in particular, to use of polypeptide-in37 and a skincare product containing the polypeptide-in37.

### BACKGROUND

Sensitive skin has affected people's quality of life to a great extent. Sensitive skin typically exhibits allergic symptoms and discomforts such as redness, itching, dryness, stinging, burning and tightness. Although identifying specific allergens is often challenging, the shared underlying pathology is "damaged skin barrier", where the skin surface loses its normal protective function, leading to moisture loss. This results in dryness, flaking or itching of the skin. Meanwhile, allergens such as pathogens, pollen, and chemicals on the skin surface can easily penetrate the damaged skin barrier into the skin, further triggering skin inflammatory reactions.

However, most of the existing skincare products for alleviating skin sensitivity problems rely on added pharmaceutical agents. These pharmaceutical agents present limitations during use including dosage challenges, potential adverse reactions in certain populations, and suboptimal efficacy. Therefore, they are unsuitable for widespread integration into daily-use cosmetical and skincare products.

Polypeptides, compounds formed by amino acids linked via peptide bonds, serve as intermediate products of protein hydrolysis. They participate extensively in human biological processes activities and represent substances with well-defined efficacy, moderate activity, clear structure, and reliable safety profiles. Due to their high safety and potent bioactivity, an increasing number of polypeptides are being widely used in the development of cosmetics.

The application PCT/RU2018/000037 filed by the applicant on January 29, 2018 entered the Chinese national phase on May 22, 2019 with an application number of 201880003993.4, and was granted on June 2, 2023. That application discloses a total of 16 polypeptide molecules, all of which have antibacterial and antiviral effects. The polypeptide-in37 of the present application is the sixth molecular formula sequence disclosed therein.

### SUMMARY

One of the objectives of the present disclosure is to provide use of polypeptide-in37 in any one of the following (1)-(3):

(1) preparation of a composition with skin repairing efficacy;

(2) preparation of a composition with skin soothing efficacy; and

(3) preparation of a cream formulation, an emulsion, a gel, an essence, a toner, a shampoo, a shower gel, a facial mask, and a lyophilized product.

The polypeptide-in37 has a molecular formula as follows:

$$\text{H-Ile-Leu-Pro-D-Phe-Lys-D-Phe-Pro-D-Phe-D-Phe-Pro-D-Phe-Arg-Arg-NH}_2.$$

The polypeptide-in37 has a sequence of ILPFKFPFFPFRR-$NH_2$ (SEQ ID NO:1), and F is D-phenylalanine (D-Phe).

Polypeptide-in37, as a chemically synthesized polypeptide, has a purity of more than 99% obtained by the current synthesis technology. The polypeptide-in37 is independently developed by the applicant, which is composed of 13 amino acids with a molecular weight of 1,711Da (below 2,000 Da), making it suitable for skin absorption.

After a large number of experiments, the applicant has discovered that polypeptide- in37 exhibits novel efficacy. When applied to the skin, it demonstrates safety and excellent skin repairing and skin soothing functions. The results from a large number of efficacy tests and toxicology tests are satisfactory. Its utilization as an active ingredient in cosmetic formulations holds significant commercial potential.

In view of this, we have filed for patent protection in the field of cosmetic ingredients as protection for future commercial development of products.

The specific molecular structure is as follows:

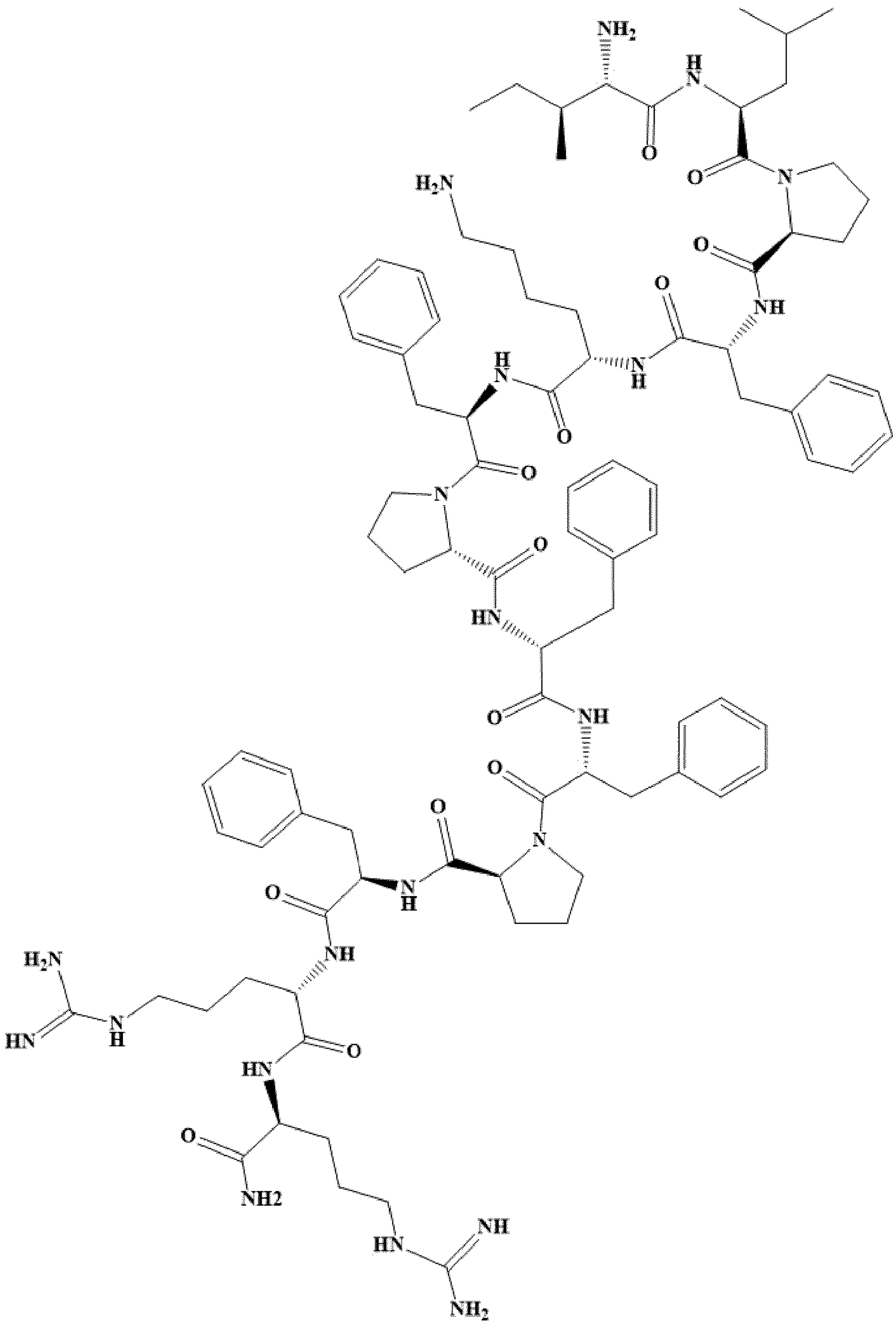

Ile-Leu-Pro-DPhe-Lys-DPhe-Pro-DPhe-DPhe-Pro-DPhe-Arg-Arg-$NH_2$.

Preferably, the polypeptide-in37 has a concentration of 0-0.0391 mg/mL, excluding 0.

Preferably, in use of (1) preparation of a composition with skin repairing efficacy, the polypeptide-in37 has a concentration of 0.0156-0.0313 mg/mL.

Preferably, in use of (2) preparation of a composition with skin soothing efficacy, the polypeptide-in37 has a concentration of 0.00049-0.0391 mg/mL.

Preferably, in use of (3) preparation of a cream product, an emulsion, a gel, an essence, a toner, a shampoo, a shower gel, a facial mask, and a lyophilized product, the polypeptide-in37 is in a form including a granular form, a powdery form, or a water-soluble liquid form.

Provided is a skincare product, wherein a raw material of the skincare product comprises polyeptide-in37, accounting for 0.005%-2% of a total mass of the skincare product.

Preferably, the skincare product further comprises aqueous phase components, an oil phase component and a

thickening agent.

The aqueous phase components comprise polypeptide-in37, a water-soluble active ingredient, glycerin, sorbitol, hydroxyethyl cellulose and purified water. The water-soluble active ingredient is at least one selected from the group consisting of hyaluronic acid, ceramides, collagen, niacinamide, carnosine, acetyl hexapeptide, acetyl tetrapeptide, hydroxypropyl tetrahydropyrantriol and sodium alginate.

The collagen is a water-soluble collagen with a molecular weight of 6,000, of Analytical Reagent (AR) grade, purchased from a Biotechnology Co., Ltd in Sichuan Province, China.

The oil phase component comprises at least one selected from the group consisting of olive oil, almond oil, shea butter, coconut oil, jojoba oil, lanolin, avocado oil, and vitamin E.

The thickening agent comprises at least one selected from the group consisting of spermaceti wax, potassium laurate and cocoa butter.

Preferably, the raw materials of the above-mentioned skincare product comprise the following components by mass percentage: polypeptide-in37 0.005%-2%, water-soluble active ingredient 0.01%-18%, glycerol 1%-20%, sorbitol 1%-20%, hydroxyethyl cellulose 1%-20%, oil phase component 0.1%-80%, thickening agent 0.1%-30%, and the balance being water.

Preferably, the skincare product is at least one selected from the group consisting of a cream formulation, an emulsion, a gel, an essence, a toner, a shampoo, a shower gel, a facial mask, and a lyophilized product.

Provided is a method for preparing the above-mentioned skincare product, which comprises the following steps:

(1) adding the aqueous phase components polypeptide-in37, water-soluble active ingredient, glycerol, sorbitol and hydroxyethyl cellulose into purified water, and heating a resulting mixture under stirring at 40-80 °C for 2-8 h;

(2) heating the oil phase component under stirring at 40-80 °C for 2-8 h;

(3) under continuous stirring of the aqueous phase components, adding the oil phase component into the aqueous phase components to allow mixing and emulsification until the two phases are completely emulsified; and

(4) adding the thickening agent to continue mixing and emulsification under heating and stirring at 40-8°C for 2-8 h, and then allowing to stand for 1-5 h for shaping and stabilization.

In the present application, the known polypeptide-in37 is further studied and its novel applications were found. Specifically, the polypeptide-in37 exhibits skin repairing and soothing functions, making it suitable for use in the preparation of a composition with skin repairing efficacy and/or skin soothing efficacy, and also suitable for preparing cream formulations, emulsions, gels, essences, toners, shampoos, shower gels, facial masks, and lyophilized products.

## BRIEF DESCRIPTION OF DRAWINGS

Fig. 1 is a diagram showing a comparison of cell viability of each group in Example 2.

Fig. 2 is a diagram showing a comparison of cell healing rate of each group in Example 2.

Fig. 3 is a photomicrograph showing a comparison of cell migration of each group in Example 2.

Fig. 4 is a diagram showing a comparison of loricrin (LOR) immunofluorescence staining results of each group in Example 3.

Fig. 5 is a diagram showing a comparison of filaggrin (FLG) immunofluorescence staining results of each group in Example 3.

Fig. 6 is a diagram showing a comparison of relative integrated optical density (IOD) values of LOR of each group in Example 3.

Fig. 7 is a diagram showing a comparison of relative integrated optical density (IOD) values of FLG of each group in Example 3.

Fig. 8 is a diagram showing a comparison of cell viability of each group in Example 4.

Fig. 9 is a diagram showing a comparison of mast cell degranulation rates of each group in Example 4.

Fig. 10 is a photomicrograph showing a morphological comparison of mast cell degranulation of each group in Example 4.

Fig. 11 is a diagram showing a comparison of cell viability of each group in Example 5.

Fig. 12 is a diagram showing a comparison of TNF-$\alpha$ content of each group in Example 5.

Fig. 13 is a diagram showing a comparison of IL-6 content of each group in Example 5.

Fig. 14 is a diagram showing a comparison of IL-1$\alpha$ content of each group in Example 5.

In the above figures, the significance of the NC group compared with the BC group is indicated by #, $P < 0.05$ is indicated by #, and P < 0.01 is indicated by ##; and when compared with the NC group, the significance of the PC group and the sample group is expressed as *, $P < 0.05$ is expressed as *, and $P < 0.01$ is expressed as **.

**DETAILED DESCRIPTION**

The present disclosure will be further explained below in conjunction with specific embodiments.

Example 1 Safety Test

1. The safety test of powder polypeptide-in 37 was carried out in accordance with the *Safety and Technical Standards for Cosmetics* (2015 edition), and the results were as follows:

(1) Microbiological Testing: total bacterial count, mold count and yeast count were all <10 CFU/g, and thermotolerant coliforms, *Staphylococcus aureus* and *Pseudomonas aeruginosa* were not detected.

(2) Physicochemical Testing: a mercury content was <0.002mg/kg, a lead content was <1.5mg/kg, an arsenic content was <0.01mg/kg, and a cadmium content was <0.18mg/kg.

2. Toxicological testing was conducted in accordance with Chapter 6, Section 8 (Bacterial Reverse Mutation Assay) of the *Safety and Technical Standards for Cosmetics* (2015 edition). After detection with five experimental strains, the samples added with and without mouse liver microsomal enzyme (S9 mixture) showed negative results, so it was judged that the polypeptide-in37 was mutagenic negative.
3. The dermal irritation test was conducted in accordance with Chapter 6, Section 4 (Dermal Irritation Test) of the *Safety and Technical Standards for Cosmetics* (2015 Edition).
3.1 Experimental Animals: New Zealand White Rabbits; Grade: Conventional; Quantity: 4; Sex: Female; Weight Range: 2.158kg-2.339kg; Animal Origin: Shanghai Songjiang Chedun Laboratory Animal Breeding Co., Ltd., China; Experimental Animal Production License No.: SCXK (Shanghai) 2022-0001, Quality Certificate number: 20220001001735.
3.2 Housing Conditions: Conventional animal room (room number: 322), breeding room temperature 16-26 °C, relative humidity 40%-70%, experimental animal use license number: SYXK (Shanghai)2021-0023.
3.3 Feed Supplier: Shanghai Zhouyu Biotechnology Co., Ltd., China; production license number: Shanghai Feed Certificate (2021) 04027. Water: Ad libitum.
3.4 Test Methodology

3.4.1 Before the experiment, the animals were acclimatized in the experimental animal room for 3 days to ensure the health and intact skin of the experimental animals.

3.4.2 About 24 h before the test, the hair on both sides of the spine of the experimental animals were shaved off. The shaved area was about 3 cm $\times$ 3 cm on the left and right sides. The skin should not be damaged. The application area was 2.5 cm $\times$ 2.5 cm.

3.4.3 The next day, after weighing, 0.5 g of the test substance (polypeptide-in37) was fully moistened with distilled water and applied to left shaved areas of the experimental animals, covered with two layers of gauze (2.5 cm $\times$ 2.5 cm) and a layer of cellophane, and then secured with hypoallergenic tape and bandage. The right shaved area served as blank control. The test substance (polypeptide-in37) was applied once a day for 14 consecutive days.

3.4.4 Clinical Observation: Starting from the next day, the remaining test substance was removed with warm water, and the results were observed after 1 h.

3.4.5 Judgement Criteria and Scoring: The evaluation was conducted in accordance with Part 5.4.3 (Result Assessment) of Chapter 6, Section 4 (Dermal Irritation/Corrosion Test) in the *Safety and Technical Standards for Cosmetics* (2015 Edition). Cumulative dermal irritation responses were scored through daily observation and grading of erythema and edema in both test and control groups. The mean irritation score per animal per day was calculated to determine dermal irritation potential.

3.5 Test Results

Table 1-1 Results of Dermal Irritation Test in New Zealand White Rabbits

| Application Day | n | Dermal Irritation Score | | | | | |
|---|---|---|---|---|---|---|---|
| | | Test Substance | | | Control | | |
| | | Erythema | Edema | Total Score | Erythema | Edema | Total Score |
| 1 | 4 | 0 | 0 | 0 | 0 | 0 | 0 |
| 2 | 4 | 0 | 0 | 0 | 0 | 0 | 0 |
| 3 | 4 | 0 | 0 | 0 | 0 | 0 | 0 |
| 4 | 4 | 0 | 0 | 0 | 0 | 0 | 0 |
| 5 | 4 | 0 | 0 | 0 | 0 | 0 | 0 |
| 6 | 4 | 0 | 0 | 0 | 0 | 0 | 0 |
| 7 | 4 | 0 | 0 | 0 | 0 | 0 | 0 |
| 8 | 4 | 0 | 0 | 0 | 0 | 0 | 0 |
| 9 | 4 | 0 | 0 | 0 | 0 | 0 | 0 |
| 10 | 4 | 0 | 0 | 0 | 0 | 0 | 0 |
| 11 | 4 | 0 | 0 | 0 | 0 | 0 | 0 |
| 12 | 4 | 0 | 0 | 0 | 0 | 0 | 0 |
| 13 | 4 | 0 | 0 | 0 | 0 | 0 | 0 |
| 14 | 4 | 0 | 0 | 0 | 0 | 0 | 0 |
| Cumulative Mean Score Per Animal (14-day) | | 0.00 | | | 0.00 | | |
| Daily Mean Score per Animal | | 0.00 | | | 0.00 | | |
| Note: Daily Mean Score per Animal (dermal irritation index) = Total cumulative scores of erythema and edema per animal over 14 days / (number of test animals × test days). | | | | | | | |

In summary, polypeptide-in37 demonstrated non-irritating potential in repeated dermal irritation studies on New Zealand White rabbits.

4. Skin sensitization test was performed according to Chapter 6, Section 6 (Skin Sensitization Test) of *the Safety and Technical Standards for Cosmetics* (2015 Edition).

1-Chloro-2,4-dinitrobenzene (manufacturer: Tokyo Chemical Industry Co., Ltd., Lot No. WT6CA-LI) was used as a positive control. Acetone (manufacturer: Sinopharm Chemical Reagent Co., Ltd., China, Lot No. 20211022) was used as a vehicle solvent. An induction concentration of the positive control was 0.8% (w/v), and a challenge concentration was 0.2% (w/v) with both administered at 0.2 mL per animal.

4.1. Experimental Animals: Albino guinea pigs; Grade: Conventional; Group size: Test substance Group: 20, Negative control group: 10, and Positive control group: 20; Weight Range: 224-295 g; Animal Origin: Shanghai Songjiang Chedun Laboratory Animal Breeding Co., Ltd., China; Experimental Animal Production License No.: SCXK (Shanghai) 2022-0001, Quality Certificate number: 20220001001734.

4.2 Housing Conditions: Conventional animal room (room number: 327), breeding room temperature 18 °C-29

°C, relative humidity 40%-70%, and experimental animal usage license number: SYXK(Shanghai)2021-0023.

4.3 Feed Supplier: Wuhan Wanqian Jiaxing Biotechnology Co., Ltd., production license number: SCXK(E) 2021-0011. Water: Ad libitum.

4.4. Bedding Supplier: Pizhou Xiaohe Technology Development Co., Ltd., China; production license number: Su Shiyu (2021) 03006.

4.5 Test Methodology

4.5.1 Before the experiment, the animals were acclimatized in the experimental animal room for 3 days to ensure the healthy and intact skin of the experimental animals.

4.5.2 About 24 h before the experiment, the hair on the left side of the backs of the albino guinea pigs was shaved off with a shaved area of 4-6 $cm^2$.

4.5.3 Induction Exposure: 0.2 g of the test substance (polypeptide-in37) at an induction-concentration was fully moistened with distilled water and applied to the left shaved areas ($2\times2$ $cm^2$) of the albino guinea pigs, covered with two layers of gauze and a layer of cellophane, and then secured with hypoallergenic tape for 6 h. The same method was repeated on the $7^{th}$ day and 14th day.

4.5.4 24 h before the challenge exposure, the hair on the right side of the backs of the albino guinea pigs was shaved off with a shaved area of 4-6 $cm^2$.

4.5.5 Challenge Exposure: 14 days after the last induction, 0.2 g the test substance (polypeptide-in37) at a challenge-concentration was fully moistened with distilled water and applied to the right shaved area (2 cm $\times$ 2 cm) of the experimental animals, covered with two layers of gauze and one layer of cellophane, and then secured with hypoallergenic tape for 6 h. Skin sensitization were observed at 24h and 48h after challenge exposure.

4.5.6 Negative control group: Distilled water was used as a control during induction exposure, and the test substance at a challenge-concentration was applied during challenge exposure. The operating procedures of the negative control group were the same as those of the experimental group.

4.5.7 Positive control group: During the induction exposure, a 1-chloro-2,4-dinitrobenzene solution in acetone with a concentration of 0.8% was applied, and during the challenge exposure, a 1-chloro-2,4-dinitrobenzene solution in acetone with a concentration of 0.2% was applied. The operating procedures of the positive control group were the same as those of the experimental group.

4.5.8 Clinical Observation: Skin sensitization was observed at 24h and 48h after challenge exposure.

4.5.9 Judgment Criteria and Scoring: Skin sensitization scoring was performed according to the evaluation method of Part 5.1 (Buehler Test, BT) of Chapter 6, Section 6 (Skin sensitization test) of *Safety and Technical Standards for Cosmetics* (2015). When the sensitization score of the test substance group was ≥2, the animal was judged to have a positive skin sensitization reaction, and the sensitization intensity grade of the test substance was obtained according to the sensitization intensity comparison table. When the sensitization rate was 0%, it can be judged that no skin sensitization reaction was observed.

4.6 Test Results

Table 1-2 Skin Sensitization Test Results in Albino Guinea Pigs (Buehler Test Method)

| Group | | | | | | | | | | Induction dose | Challenge dose | Observation time | Skin sensitization intensity[2] | | | Num ber | Sensit izatio |
|---|---|---|---|---|---|---|---|---|---|---|---|---|---|---|---|---|---|
| | | | | | | | | | | | | | Erythema | Edema | | | |
| | | | | | | | | | | | | | | | | | |
| | Number of animals | Initial weight[1] (g) | Final weight[1] (g) | | | | 0 | 1 | 2 | 3 | 4 | 0 | 1 | 2 | 3 | of animals with score ≥ 2 | n rate (%) |
| Negative control group | 10 | 264.40 ±20.55 | 329.90 ±15.75 | Distilled water | Original sample | 24h | 10 | 0 | 0 | 0 | 0 | 10 | 0 | 0 | 0 | 0 | 0 |
| | | | | | | | / | / | / | / | / | / | / | / | / | | |
| | | | | | | | 10 | 10 | 10 | 10 | 10 | 10 | 10 | 10 | 10 | | |
| | | | | | | 48h | 10 | 0 | 0 | 0 | 0 | 10 | 0 | 0 | 0 | 0 | 0 |
| | | | | | | | / | / | / | / | / | / | / | / | / | | |
| | | | | | | | 10 | 10 | 10 | 10 | 10 | 10 | 10 | 10 | 10 | | |
| Test substance group | 20 | 250.45 ±21.74 | 343.05 ±23.27 | Original sample | Original sample | 24h | 20 | 0 | 0 | 0 | 0 | 20 | 0 | 0 | 0 | 0 | 0 |
| | | | | | | | / | / | / | / | / | / | / | / | / | | |
| | | | | | | | 20 | 20 | 20 | 20 | 20 | 20 | 20 | 20 | 20 | | |
| | | | | | | 48h | 20 | 0 | 0 | 0 | 0 | 20 | 0 | 0 | 0 | 0 | 0 |
| | | | | | | | / | / | / | / | / | / | / | / | / | | |
| | | | | | | | 20 | 20 | 20 | 20 | 20 | 20 | 20 | 20 | 20 | | |

(continued)

| | Number of animals | Initial weight[1] (g) | Final weight[1] (g) | | | | 0 | 1 | 2 | 3 | 4 | 0 | 1 | 2 | 3 | of animals with score ≥ 2 | n rate (%) |
|---|---|---|---|---|---|---|---|---|---|---|---|---|---|---|---|---|---|
| Positive control group | 20 | 253.10 ±19.83 | 346.20 ±22.42 | 0.8% | 0.2% | 24h | 0 | 6 | 14 | 0 | 0 | 9 | 11 | 0 | 0 | 14 | 70 |
| | | | | | | | / | / | / | / | / | / | / | / | / | | |
| | | | | | | | 20 | 20 | 20 | 20 | 20 | 20 | 20 | 20 | 20 | | |
| | | | | | | 48h | 0 | 6 | 14 | 0 | 0 | 9 | 11 | 0 | 0 | 14 | 70 |
| | | | | | | | | / | / | / | / | / | / | / | / | | |
| | | | | | | | 20 | 20 | 20 | 20 | 20 | 20 | 20 | 20 | 20 | | |

Note [1]: initial and final body weights were expressed as mean ± SD.
Note [2]: Skin sensitization intensity values represent the proportion of animals exhibiting each score (0-4) relative to total test animals.
Positive control test period: December 14, 2023 to January 13, 2024.

In summary, Polypeptide-in37 demonstrated no observable skin sensitization in albino guinea pigs per the Buehler test.

Example 2 Repairing Efficacy Test-Keratinocyte Migration Assay

1. Objective: To evaluate the skin barrier repairing efficacy of test samples by assessing their effects on cell migration using human immortalized keratinocytes (HaCaT) as the model system.
The human immortalized keratinocytes (HaCaT) used in this example were purchased from Shanghai Zhongqiao Xinzhou Biotechnology Co., Ltd., China (Catalog No: ZQ0044).

2. Key Reagents: High-glucose DMEM culture medium (Gibco), fetal bovine serum (Gibco), PBS (VivaCell), MTT (Sigma), trypsin (Gibco), and DMSO (Sinopharm).

3. Major Equipment: $CO_2$ incubator (Thermo, Model 160i), biosafety cabinet (ESCO, Model LA2-6A1), inverted fluorescence microscope (Keyence BZ-X810), and microplate reader (Tecan, Spark).

4. Test Methodology

4.1. Cytotoxicity Assessment

(1) Cell Inoculation: Cells were inoculated in a 96-well plate at at inoculation density of $1\times10^4$ cells/well and incubated overnight in an incubator (37°C, 5% $CO_2$).
(2) Experimental Grouping: blank control group, solvent control group, positive control and sample group. In the sample group, 8 concentration gradients were set for each test sample, and 3 replicate wells were set under each concentration gradient.
(3) Solution Preparation: working solutions were prepared according to concentrations specified in Table 2-1.

Table 2-1 Test Concentration

| Sample | Peptide-in37 concentration gradient (%, m/V) |
|---|---|
| ① | 1.0000 |
| ② | 0.5000 |
| ③ | 0.2500 |
| ④ | 0.1250 |
| ⑤ | 0.0625 |
| ⑥ | 0.0313 |
| ⑦ | 0.0156 |
| ⑧ | 0.0078 |

(4) Administration: The administration was performed when cell confluency in the 96-well plate reached 40%-60%. In the solvent control group, 200 μL of culture medium containing 10% PBS was added to each well. In the positive control group, 200 μL of culture medium containing 10% DMSO was added to each well; 200 μL of culture medium containing samples of corresponding concentrations was added to each well of the sample group. In the blank control group, no cells were inoculated and only 200 μL of cell culture medium was added. After administration, the 96-well plate was placed in an incubator (37°C, 5% $CO_2$) for culture.
(5) Detection: After the cells were incubated for 24 h, the supernatant was discarded, and a medium containing 0.5 mg/mL MTT was added, and the cells were incubated at 37°C in the dark for 4 h. After the incubation, the supernatant was discarded, 100 μL DMSO was added to each well, and the OD value was read at 490 nm.
(6) Cell Viability Calculation: The cell viability calculation was calculated according to the below formula:

Cell viability = (OD of sample group - OD of blank control group) ÷ (OD of solvent control group - OD of blank control group) × 100%

(7) Test Results

Eight sample administration concentrations were set, and the cytotoxicity detection experiments were carried out. The MTT detection results were shown in Table 2-2.

Table 2-2MTT Detection Results

| Group | Sample concentration (%, m/V) | Cell viability (%) | |
|---|---|---|---|
| | | Mean | SD |
| Solvent control group | 1 | 100.00 | 3.24 |
| Positive control group | 10%DMSO | 16.15 | 0.31 |
| ① | 1.0000 | 45.12 | 6.73 |
| ② | 0.5000 | 69.56 | 11.13 |
| ③ | 0.2500 | 43.43 | 0.34 |
| ④ | 0.1250 | 69.89 | 0.24 |
| ⑤ | 0.0625 | 71.53 | 2.12 |
| ⑥ | 0.0313 | 82.83 | 6.76 |
| ⑦ | 0.0156 | 77.38 | 1.42 |
| ⑧ | 0.0078 | 85.21 | 1.80 |

A cell viability graph (as shown in Fig. 1) was drawn with the eight concentrations of the sample as the abscissa and the cell viability values as the ordinate.

According to the MTT results, polypeptide-in37 showed no keratinocyte toxicity within the concentration range of 0.0313% (m/V).

4.2 Cell Migration Assay

(1) Cells in the logarithmic growth phase were collected and inoculated in a 24-well culture plate at a cell density of $1.5\times10^5$ cells/well.

(2) After culturing in an incubator (37°C, 5% $CO_2$) for 24 h, a straight scratch was created perpendicularly across each well using a sterile 200 μL pipette tip, and the cells were then washed once with PBS to remove the scratched cells.

(3) Treatments were applied according to Table 2-3. The plates were incubated (37°C, 5% $CO_2$) for 24 h, with 3 parallel wells set up for each group.

Table 2-3 Experimental Design

| Group | Sample concentration (%, m/V) | Culture condition |
|---|---|---|
| Blank control (BC) | / | FBS-free medium |
| Positive Control (PC) | / | Medium containing 10% FBS |
| Polypeptide-in37 | 0.0078 | FBS-free medium |
| | 0.0156 | |
| | 0.0313 | |

(4) The migration of cells of each group were photographed using an inverted microscope, and the average scratch area was calculated using ImageProPlus software.

$$\text{Wound healing rate (\%)} = \text{(initial scratch area - current scratch area) / initial scratch area} \times 100\%$$

(5) Test Results

The test results were shown in Fig. 3, and the results analysis was shown in Tables 2-4 and Fig. 2.

Table 2-4 Summary of Cell Wound Healing Rate Results

| Group | Wound healing rate (%) | SD | P-value |
|---|---|---|---|
| Blank control (BC) | 24.10 | 2.11 | / |
| Positive Control (PC) | 60.33 | 3.25 | 0.000** |
| Polypeptide-in37 0.0078% | 26.64 | 1.11 | 0.138 |
| Ppolypeptide-in37 0.0156% | 34.63 | 0.95 | 0.001** |
| Polypeptide-in37 0.0313% | 38.21 | 0.70 | 0.000** |
| Note: when the *t*-test two-tailed test method was used for statistical analysis, the significance of the sample group and the PC group when compared with the BC group was indicated by *, $P < 0.05$ was indicated by *, and $P < 0.01$ was indicated by **. | | | |

From the above test results and analysis, it can be known that: in the cell migration assay, the cell healing level in the PC group was significantly higher than that in the BC group ($P < 0.01$), indicating that the positive control test was effective. Compared with the BC group, the cell healing rate was significantly increased after treatment with samples at concentrations of 0.0156% and 0.0313% (m/V) ($P < 0.01$).

In summary, based on the human immortalized keratinocyte (HaCaT) model, the cell healing rate of keratinocytes was significantly improved after 24 h of treatment with polypeptide-in37 at concentrations of 0.0156% and 0.0313% (m/V), and there was a statistical difference compared with the BC group ($P < 0.01$). It is confirmed that polypeptide-in37 can promote the cell healing of keratinocytes, demonstrating its repairing efficacy.

Example 3 Repairing efficacy test: Detection of Barrier-Related Protein Content in ultraviolet radiation b (UVB)- Induced Keratinocytes

1. Purpose: This study employed Human immortalized keratinocytes (HaCaT) to evaluate the repairing efficacy of the sample by detecting the protein content of loricrin (LOR) and filaggrin (FLG) using immunofluorescence technology. The HaCaT cells were purchased from Shanghai Zhongqiao Xinzhou Biotechnology Co., Ltd., China (Catalog No.: ZQ0044).
2. Key Reagents: High-glucose DMEM culture medium (Gibco), PBS (Gibco), trypsin (Gibco), MTT (sigma), LOR antibody (Abcam), FLG antibody (Proteintech), goat anti-rabbit secondary antibody (Proteintech), BSA (Abcam), and DAPI stain.
3. Key Equipment: $CO_2$ incubator (Thermo, Model 160i), 24-well plate slide (Solabo), ultraviolet phototherapy device (SIGMA, Model SS-03), biosafety cabinet (Sujing Antai, BSC-1604IIA2), inverted fluorescence microscope (Keyence BZ-X810), and microplate reader (Tecan, Model Spark).
4. Methodology (immunofluorescence test)
(1) Cell Inoculation: The sterile cell slide was placed in the 24-well plate. Cells were inoculated in the 24-well plate at an optimal density ($1\times10^5$ cells/well) and incubated overnight (37°C, 5% $CO_2$).
(2) Experimental Grouping: the experiment set up blank control group, negative control group, positive control group and sample group, and the sample group had multiple concentration gradients.

Table 3-1 Experimental Design

| Group | Sample concentration (%, mV) | Modeling conditions |
|---|---|---|
| Blank control (BC) | / | / |
| Negative control (NC) | / | UVB 300 mJ/cm$^2$ |
| Positive Control (PC) | 100 μg/mL VC +7 μg/mL VE | |
| Polypeptide-in37 | 0.0078 | |
| | 0.0156 | |
| | 0.0313 | |

(3) Solution Preparation: Working solutions of the test substance at different concentrations were prepared using cell culture medium, according to the concentrations specified in the experimental design (Table 3-1).
(4) UVB Irradiation: Irradiation was performed when cell confluency in the 24-well plate reached 40%-60%. The negative control group, positive control group, and sample group received a total UVB radiation dose of 300 mJ/cm$^2$. The blank

control group was placed under identical conditions (UVB radiation dose: 0 J/cm$^2$).
(5) Administration: after the irradiation, the original cell culture medium was aspirated thoroughly and then treatments were administered according to different groups. In the sample group, 1.0 mL of sample working solution was added to each well, with 3 replicate wells for each concentration of working solution. In the blank control group (BC group), 1.0 mL of cell culture solution was added to each well, with 3 replicate wells. Plates were incubated (37°C, 5% $CO_2$) for 24 h.
(6) Sample Collection: supernatants were discarded and cells were washed three times with PBS.
(7) Immunofluorescence staining: routine immunofluorescence staining was performed. The main steps included: fixation, blocking, incubation with primary antibody, incubation with secondary antibody, counterstaining with DAPI, mounting, and imaging stained cells using a fluorescence microscope.
(8) The fluorescence intensity of LOR and FLG was quantitatively analyzed using ImageProPlus software.
5. Test Results

5.1 LOR Test Results

Immunofluorescence staining results of LOR protein in keratinocytes after treating with the sample were shown in Fig.4 (blue fluorescence: nuclei; green fluorescence: LOR. Expression levels were proportional to the area and intensity of green fluorescence-larger areas and deeper color indicated higher LOR content). The relative IOD values (i.e., integrated optical density, reflecting the relative content of LOR) were shown in Table 3-2 and Fig. 6.

Table 3-2 Summary of LOR Immunofluorescence Analysis Results

| Group | Relative IOD (Mean) | SD | P-value |
|---|---|---|---|
| Blank control (BC) | 1.00 | 0.08 | / |
| Negative control (NC) | 0.55 | 0.01 | 0.001### |
| Positive Control (PC) | 1.01 | 0.02 | 0.000** |
| Polypeptide-in37 0.0078% | 0.73 | 0.05 | 0.005** |
| Polypeptide-in37 0.0156% | 0.82 | 0.06 | 0.002** |
| Polypeptide-in37 0.0313% | 1.05 | 0.01 | 0.000** |
| Note: when the *t*-test two-tailed test method was used for statistical analysis, the significance of the NC group compared with the BC group was indicated by #, $P < 0.05$ was indicated by #, and P < 0.01 was indicated by ##. Compared with the NC group, the PC group and the sample group were expressed as * for significance, $P < 0.05$ was expressed as *, and $P < 0.01$ was expressed as **. | | | |

Compared with the BC group, the LOR content of the NC group was significantly decreased ($P < 0.01$), indicating that the UVB-induced modeling was successful in this experiment. Compared with the NC group, the LOR content in the PC group was significantly increased at the administration concentration of 100 μg/mL VC and 7 μg/mL VE (P<0.01), indicating that the positive control of this experiment was effective.
Compared with the NC group, the LOR content of polypeptide-in37 at the administration concentrations of 0.0078%, 0.0156% and 0.0313% (m/V) was significantly increased ($P < 0.01$).

5.2. FLG Test Results

Immunofluorescence staining results of FLG in keratinocytes after treatment with the sample were shown in Fig.5 (blue fluorescence: nuclei; green fluorescence: FLG. Expression levels were proportional to the area and intensity of green fluorescence-larger areas and deeper color indicated higher FLG content). Relative IOD values (Integrated Optical Density, representing relative FLG content) were presented in Table 3-3 and Fig. 7.

Table 3-3 Summary of FLG Immunofluorescence Analysis Results

| **Group** | **Relative IOD (Mean)** | **SD** | **P-value** |
|---|---|---|---|
| Blank control (BC) | 1.00 | 0.02 | / |
| Negative control (NC) | 0.52 | 0.02 | 0.000### |
| Positive Control (PC) | 0.90 | 0.01 | 0.000** |
| Polypeptide-in37 0.0078% | 0.70 | 0.02 | 0.000** |

(continued)

| Group | Relative IOD (Mean) | SD | P-value |
|---|---|---|---|
| Polypeptide-in37 0.0156% | 0.88 | 0.02 | 0.000** |
| Polypeptide-in37 0.0313% | 0.93 | 0.02 | 0.000** |
| Note: when the *t*-test two-tailed test method was used for statistical analysis, the significance of the NC group compared with the BC group was indicated by #, $P < 0.05$ was indicated by #, and $P < 0.01$ was indicated by ##; compared with the NC group, the PC group and the sample group were expressed as * for significance, $P < 0.05$ was expressed as *, and $P < 0.01$ was expressed as **. | | | |

Compared with the BC group, the FLG content of the NC group was significantly decreased ($P < 0.01$), indicating that the UVB-induced modeling was successful in this experiment. Compared with the NC group, the FLG content of the PC group was significantly increased at the administration concentration of 100 μg/mL VC and 7 μg/mL VE ($P < 0.01$), indicating that the positive control of this experiment was effective.

Compared with the NC group, the FLG content of polypeptide-in37 at the concentrations of 0.0078%, 0.0156% and 0.0313% (m/V) was significantly increased ($P < 0.01$).

In summary, based on the human immortalized keratinocyte (HaCaT) model, the content of cell barrier-related proteins loricrin (LOR) and filaggrin (FLG) of polypeptide-in37 at concentrations of 0.0078%, 0.0156% and 0.0313% (m/V) was significantly increased, which was statistically different from that of the NC group (P < 0.01). It was confirmed that polypeptide-in37 had a repairing efficacy.

Example 4 Soothing Efficacy: Detection of C48/80-Stimulated Mast Cell Degranulation

1. Purpose: To establish a degranulation model using C48/80-stimulated mouse mastocytoma P815 cells, and evaluate the soothing efficacy of test samples by testing changes in mast cell degranulation morphology and degranulation rate.
The mouse mastocytoma P815 cells were purchased from the Cell Bank of the Chinese Academy of Sciences with the catalog number TCM12.

2. Key Reagents: High-glucose DMEM culture medium (VivaCell), fetal bovine serum (Gibco), PBS (VivaCell), CCK-8 (Biyuntian), DMSO (Sinopharm), and trypsin (Gibco).

3. Key Equipment: $CO_2$ incubator (Thermo, Model 160i), biosafety cabinet (Sujing Antai, Model BSC-1604IIA2), inverted fluorescence microscope (Keyence, Model BZ-X810), and microplate reader (Tecan, Model Spark).

4. Experimental Methodology

4.1. Cytotoxicity Assay

(1) Cell Inoculation: Cells were inoculated in a 96-well plate at an inoculation density of $1\times10^4$ cells/well and incubated overnight in an incubator (37°C, 5% $CO_2$).
(2) Experimental Grouping: the experiment included black control group, solvent control group, positive control group and sample group. In the sample group, 8 concentration gradients were set for each sample, with 3 replicate wells per concentration.
(3) Solution Preparation: working solutions of test samples at different concentrations were prepared according to Table 4-1.

Table 4-1 Test Concentration

| Sample | Polypeptide-in37 concentration gradient (%, mV) |
|---|---|
| ① | 0.00391 |
| ② | 0.00195 |
| ③ | 0.00098 |

(continued)

| Sample | Polypeptide-in37 concentration gradient (%, mV) |
|---|---|
| ④ | 0.00049 |
| ⑤ | 0.00024 |
| ⑥ | 0.00012 |
| ⑦ | 0.00006 |
| ⑧ | 0.00003 |

(4) Administration: the administration was performed when cell confluency in the 96-well plate reached 40%-60%. In the solvent control group, 200 μL of culture medium containing 10% PBS was added to each well; in the positive control group, 200 μL of culture medium containing 10% DMSO was added to each well; 200 μL of culture medium containing samples of corresponding concentrations was added to each well of the sample group; in the black control group, no cells were inoculated and only 200 μL of cell culture medium was added. After administration, the 96-well plate was placed in an incubator (37°C, 5% $CO_2$) for culture.
(5) Detection: after the cells were incubated for 24 h, CCK-8 working solution was added and incubated at 37 °C in the dark for 4 h. After the incubation, the OD value was read at 450 nm.
(6) Cell viability was calculated according to the below formula:

Cell viability = (OD of sample group - OD of black control group) / (OD of solvent control group - OD of black control group) × 100%

(7) Test Results
Eight sample administration concentrations were set, and cytotoxicity detection experiments were carried out. The CCK-8 detection results were shown in Table 4-2.

Table 4-2 CCK-8 Detection Results

| Group | Sample concentration (%, mV) | Cell viability (%) | |
|---|---|---|---|
| | | Mean | SD |
| Solvent control group | 1 | 100.00 | 8.77 |
| Positive control group | 10%DMSO | 13.63 | 1.37 |
| ① | 0.00391 | 85.17 | 1.55 |
| ② | 0.00195 | 87.47 | 4.41 |
| ③ | 0.00098 | 91.16 | 1.67 |
| ④ | 0.00049 | 90.42 | 1.34 |
| ⑤ | 0.00024 | 92.95 | 3.45 |
| ⑥ | 0.00012 | 93.54 | 2.68 |
| ⑦ | 0.00006 | 92.74 | 1.98 |
| ⑧ | 0.00003 | 94.48 | 0.60 |

A cell viability graph was drawn with the eight concentrations of the sample as the abscissa and the cell viability values as the ordinate (see Fig. 8).
Therefore, according to the CCK-8 detection results, polypeptide-in37 did not show mast cell toxicity within the concentration range of 0.00391% (m/V).

4.2 P815 Cell Degranulation Assay

(1) Cell Inoculation: Cells were inoculated into a 24-well plate at a inoculation density of $1\times10^5$ cells/well and incubated overnight in an incubator (37°C, 5% $CO^2$).
(2) Solution Preparation: Working solutions of test substances with different concentrations were prepared according to

Table 4-3.

Table 4-3 Experimental Design

| Group | Sample concentration (%, mV) | Modeling conditions |
|---|---|---|
| Blank control (BC) | / | / |
| Negative control (NC) | / | C48/80 100 μg/mL |
| Positive Control (PC) | 1 mg/mL Sodium cromolyn | |
| Polypeptide-in37 | 0.00098 | |
| | 0.00195 | |
| | 0.00391 | |

(3) Administration: when the cell confluency in the 24-well plate reached 40%-50%, treatments were administered according to the experimental design in Table 4-3, with 3 replicate wells in each group. Each well received 1 mL of culture medium containing the test sample at different designated concentrations. After administration, the 24-well plate was placed in an incubator (37°C, 5% CO2) for 2 h.

(4) C48/80 Stimulation: after the samples were incubated for 2 h, supernatants were discarded. According to the experiment, 1 mL of serum-free high-glucose DMEM culture medium was added to the blank control group, 1 mL of serum-free high-glucose DMEM culture medium containing C48/80 was added to the negative control group, 1 mL of serum-free high-glucose DMEM culture medium containing C48/80 and sodium cromoglycate was added to the positive control group, and 1 mL of serum-free high-glucose DMEM culture medium containing C48/80 and sample with corresponding concentration was added to the sample group. After the administration, C48/80 stimulation was performed for 45 minutes, and the reaction was terminated in an ice bath.

(5) Cell Morphology Observation: after the culture was completed, the degranulation of cells in each group was observed under an inverted microscope and photographed. The photos were counted and the cell degranulation rate was calculated using IPP software.

Degranulation rate (%) = total number of degranulated cells ÷ (total number of degranulated cells / total number of non-degranulated cells) × 100%.

(6) Test Results

Based on the degranulation model of C48/80 stimulated mast cells, the test was carried out and the degranulation rate was calculated. The morphological results were shown in Fig. 10, and the statistical results of the degranulation rate were shown in Table 4-4 and Fig. 9.

Table 4-4 Mast Cell Degranulation Rate

| Group | Mean(%) | SD | P-value |
|---|---|---|---|
| Blank control (BC) | 0.58 | 0 | / |
| Negative control (NC) | 88.13 | 0.03 | 0.000## |
| Positive Control (PC) | 9.29 | 0.03 | 0.000** |
| Peptide-in37 0.00098% | 57.91 | 0.02 | 0.000** |
| Peptide-in37 0.00195% | 44.62 | 0.01 | 0.000** |
| Peptide-in37 0.00391% | 37.41 | 0.03 | 0.000** |
| Note: when the *t*-test method was used for statistical analysis, the significance of the NC group compared with the BC group was indicated by #, $P < 0.05$ was indicated by #, and $P < 0.01$ was indicated by ##; compared with the NC group, the PC group and the sample group were expressed as * for significance, $P < 0.05$ was expressed as *, and $P < 0.01$ was expressed as **. | | | |

Compared with the BC group, the mast cell degranulation rate in the NC group was significantly increased ($P < 0.01$), indicating that the degranulation model was successfully established in this experiment. Compared with the NC group, the mast cell degranulation rate in the PC group was significantly reduced ($P < 0.01$), indicating that the positive

control test was effective.

Compared with the NC group, the mast cell degranulation rate was significantly reduced when the concentrations of peptide-in37 were 0.00098%, 0.00195% and 0.00391% (m/V) ($P < 0.01$).

In summary, based on the C48/80-stimulated mouse mast cell tumor cell P815 model, the mast cell degranulation rate was significantly reduced when the concentration of polypeptide-in37 was 0.00098%, 0.00195% and 0.00391% (m/V), and there was a statistical difference compared with the NC group ($P < 0.01$), confirming that polypeptide-in37 had a soothing efficacy.

Example 5 Soothing Efficacy: Detection of content of inflammatory cytokines (TNF-α, IL-6, IL-1α) in LPS-stimulated RAW264.7 macrophages

1. Purpose: To evaluate the soothing efficacy of test samples by measuring changes in content of inflammatory cytokines (TNF-α, IL-6, IL-1α) secretion using an LPS-stimulated RAW264.7 macrophage model.

2. Key reagents: High-glucose DMEM culture medium (Gibco), fetal bovine serum (Gibco), PBS (VivaCell), MTT (Sigma), DMSO (Sinopharm), trypsin (Gibco), LPS (Sigma), dexamethasone (Sigma), Mouse TNF-α ELISA Kit (Boster), Mouse IL-6 Valukine ELISA Kit (Boster), Mouse IL-1α Valukine ELISA Kit (Boster).

3. Key equipment: $CO_2$ incubator (Thermo, Model 160i), biosafety cabinet (Sujing Antai, Model BSC-1604IIA2), inverted fluorescence microscope (Keyence, Model BZ-X810), and microplate reader (Tecan, Model Spark).

4. Test Methodology

4.1 Cytotoxicity Assay

(1) Cell Inoculation: Cells were inoculated in a 96-well plate at a inoculation density of $1\times10^4$ cells/well and incubated overnight in an incubator (37°C, 5% $CO_2$).

(2) Experimental Grouping: the experiment included black control group, solvent control group, positive control group and sample group. In the sample group, 8 concentration gradients were set for each sample, with 3 replicate wells per concentration.

(3) Solution Preparation: Working solutions of samples with different concentrations were prepared according to Table 5-1.

Table 5-1 Test concentration

| Sample | Polyeptide-in37 concentration gradient (%, mV) |
|---|---|
| ① | 0.00391 |
| ② | 0.00195 |
| ③ | 0.00098 |
| ④ | 0.00049 |
| ⑤ | 0.00024 |
| ⑥ | 0.00012 |
| ⑦ | 0.00006 |
| ⑧ | 0.00003 |

(4) Administration: The administration was performed when the cell confluency in the 96-well plate reached 40%-60%. In the solvent control group, 200 μL of culture medium containing 10% PBS was added to each well; in the positive control group, 200 μL of culture medium containing 10% DMSO was added to each well; 200 μL of culture medium containing samples of corresponding concentrations was added to each well of the sample group; in the black control group, no cells were inoculated and only 200 μL of cell culture medium was added. After administration, the 96-well plate was placed in an incubator (37°C, 5% $CO_2$) for culture.

(5) Detection: after the cells were incubated for 24 h, supernatants were discarded, and MTT working solution (0.5 mg/mL) was added. The cells were incubated at 37 °C in the dark for 4 h. After the incubation, the supernatants were discarded, 100 μL DMSO was added to each well, and the OD value was read at 490 nm.

(6) Calculation of Cell Viability: The cell viability was calculated according to the below formula:

Cell viability = [(OD of sample group - OD of black control group) ÷ (OD of solvent control group - OD of black control group)] × 100%

(7) Test Results

Eight sample administration concentrations were set, and cytotoxicity detection experiments were carried out. The MTT detection results were shown in Table 5-2.

Table 5-2 MTT Detection Results

| Group | Sample concentration (%, mV) | Cell viability (%) | |
|---|---|---|---|
| | | Mean | SD |
| Solvent control group | 1 | 100.00 | 6.78 |
| Positive control group | 10%DMSO | 9.25 | 0.40 |
| ① | 0.00391 | 71.50 | 4.23 |
| ② | 0.00195 | 81.96 | 2.08 |
| ③ | 0.00098 | 96.70 | 2.53 |
| ④ | 0.00049 | 99.48 | 1.70 |
| ⑤ | 0.00024 | 101.48 | 3.22 |
| ⑥ | 0.00012 | 108.71 | 2.38 |
| ⑦ | 0.00006 | 107.24 | 5.24 |
| ⑧ | 0.00003 | 104.87 | 6.08 |

A cell viability graph was drawn with the eight concentrations of the sample as the horizontal axis and the cell viability values as the vertical axis (see Fig. 11).

Therefore, according to the MTT results, polypeptide-in37 showed no toxicity to RAW264.7 macrophages within the concentration range of 0.00195% (m/V).

4.2. Inflammatory Cytokine Content Assay

(1) Cell incubation: Cells were inoculated in a 24-well plate at an inoculation density of $1 \times 10^5$ cells/well and incubated overnight in an incubator (37°C, 5% $CO_2$).

(2) Solution Preparation: Working solutions of the test substances were prepared at different concentrations according to Table 5-3.

Table 5-3 Experimental Design

| **Group** | **Sample concentration (%, mV)** | **Modeling conditions** |
|---|---|---|
| **Blank control (BC)** | / | 1 |
| **Negative control (NC)** | / | LPS 1 μg/mL |
| **Positive Control (PC)** | 100 μg/mL dexamethasone | |
| Polypeptide-in37 | 0.00049 | |
| | 0.00098 | |
| | 0.00195 | |

(3) Induction and Administration: When the cell confluency in the 24-well plate reached 40%-60%, 100 μL of 10×LPS working solution was added to each of the well plate according to the experimental design, the well plate was shaked left and right to ensure even distribution of the solution in the well plate, and the test sample was added. The final concentration of LPS was 1 μg/mL, with 3 replicate wells for each group. After administration, the cells were placed in an incubator (37°C, 5% $CO_2$) and cultured for 24 h.

(4) Sample Collection: After the incubation, the cell culture supernatants were collected into an EP tube (Note: the amount of samples collected depended on the detection requirements). After collection, the samples were placed in a -80 °C refrigerator for storage.
(5) TNF-α Content Detection: The detection was performed according to the instruction of the Mouse TNF-α ELISA kit.

IL-6 Content Detection: The detection was performed according to the instruction of the Mouse IL-6 ELISA kit.

IL-1α Content Detection: The detection was performed according to the instruction of Mouse IL-1α ELISA kit.

(6) Test Results
1) Based on the experimental protocol, the cell supernatants were collected and the TNF-α content was detected. The test results were shown in Table 5-4, and the change trend was shown in Fig. 12.

Table 5-4 Summary of TNF-α Data

| **Group** | **Average concentration (pg/mL)** | **SD** | **P-value** |
|---|---|---|---|
| Blank control (BC) | 473.99 | 16.07 | / |
| Negative control (NC) | 720.77 | 24.68 | 0.000# |
| Positive Control (PC) | 443.67 | 21.37 | 0.000** |
| Peptide-in37 0.00049% | 668.08 | 7.43 | 0.045* |
| Peptide-in37 0.00098% | 657.14 | 5.53 | 0.024* |
| Peptide-in37 0.00195% | 582.22 | 20.89 | 0.004** |
| Note: when the *t*-test method was used for statistical analysis, the significance of the NC group compared with the BC group was indicated by #, $P < 0.05$ was indicated by #, and $P < 0.01$ was indicated by ##; compared with the NC group, the PC group and the sample group were expressed as * for significance, $P < 0.05$ was expressed as *, and $P < 0.01$ was expressed as **. | | | |

Compared with the BC group, the secretion of inflammatory factor TNF-α by the RAW264.7 macrophages in the NC group was significantly increased ($P < 0.01$), indicating that the LPS-stimulated model was successful in this experiment. Compared with the NC group, the secretion of inflammatory factor TNF-α by the RAW264.7 macrophages in the PC group was significantly reduced under 100 μg/mL dexamethasone ($P < 0.01$), confirming the validity of the positive control test.

Compared with the NC group, the secretion of inflammatory factor TNF-α by the RAW264.7 macrophages was significantly reduced when the concentration of polypeptide-in37 was 0.00049%, 0.00098% and 0.00195% (m/V) ($P < 0.05$).

2) Based on the experimental protocol, the cell supernatants were collected for detection of the IL-6 content. The test results were shown in Table 5-5, and the change trend was shown in Fig. 13.

Table 5-5 Summary of IL-6 Data

| Group | Mean concentration (pg/mL) | SD | P-value |
|---|---|---|---|
| Blank control (BC) | 38.96 | 0.46 | / |
| Negative control (NC) | 76.07 | 2.36 | 0.000### |
| Positive Control (PC) | 47.56 | 0.65 | 0.000** |
| Polypeptide-in37 0.00049% | 51.50 | 2.61 | 0.001** |
| Polypeptide-in37 0.00098% | 50.72 | 2.63 | 0.001** |
| Polypeptide-in37 0.00195% | 48.76 | 1.19 | 0.000** |
| Note: when the *t*-test method was used for statistical analysis, the significance of the NC group compared with the BC group was indicated by #, $P < 0.05$ was indicated by #, and $P < 0.01$ was indicated by ##; compared with the NC group, the PC group and the sample group were expressed as * for significance, $P < 0.05$ was expressed as *, and $P < 0.01$ was expressed as **. | | | |

Compared with the BC group, the secretion of inflammatory factor IL-6 by the RAW264.7 macrophages in the NC group was significantly increased ($P < 0.01$), indicating that the LPS-stimulated model was successful in this experiment. Compared with the NC group, the secretion of inflammatory factor IL-6 by the RAW264.7 macrophages in the PC group was significantly reduced under 100 μg/mL dexamethasone ($P < 0.01$), confirming the validity of the positive control test.
Compared with the NC group, the secretion of inflammatory factor IL-6 by the RAW264.7 macrophages was significantly reduced when the concentration of peptide-in37 was 0.00049%, 0.00098% and 0.00195% (m/V) ($P < 0.01$).
3) Based on the experimental protocol, the cell supernatants were collected for detection of the IL-1α content. The test results were shown in Tables 5-6, and the change trend wasa shown in Fig. 14.

Table 5-6 Summary of IL-1α Data

| Group | Mean concentration (pg/mL) | SD | P-value |
|---|---|---|---|
| Blank control (BC) | 5.53 | 0.33 | / |
| Negative control (NC) | 10.45 | 0.19 | 0.000### |
| Positive Control (PC) | 6.29 | 0.23 | 0.000** |
| Peptide-in37 0.00049% | 9.00 | 0.79 | 0.064 |
| Peptide-in37 0.00098% | 7.38 | 0.67 | 0.003** |
| Peptide-in37 0.00195% | 6.85 | 0.46 | 0.001** |
| Note: when the *t*-test method was used for statistical analysis, the significance of the NC group compared with the BC group was indicated by #, $P < 0.05$ was indicated by #, and $P < 0.01$ was indicated by ##; compared with the NC group, the PC group and the sample group were expressed as * for significance, $P < 0.05$ was expressed as *, and $P < 0.01$ was expressed as **. | | | |

Compared with the BC group, the secretion of inflammatory factor IL-1α by the RAW264.7 macrophages in the NC group was significantly increased ($P < 0.01$), indicating that the LPS-stimulated model was successful in this experiment. Compared with the NC group, the secretion of inflammatory factor IL-1α by the RAW264.7 macrophages in the PC group was significantly reduced under 100 μg/mL dexamethasone ($P < 0.01$), confirming the validity of the positive control test.
Compared with the NC group, the secretion of the inflammatory factor IL-1α by the RAW264.7 macrophages was significantly reduced when the concentration of peptide-in37 was 0.00098% and 0.00195% (m/V) ($P < 0.01$).
In summary, based on the LPS-stimulated RAW264.7 macrophage model, when the concentration of polypeptide-in37 was 0.00049%, 0.00098% and 0.00195% (m/V), the secretion of inflammatory factors TNF-α and IL-6 in the RAW264.7 macrophages was significantly reduced, and there was a statistical difference compared with the NC group ($P < 0.05$). When the polypeptide-in37 was at concentrations of 0.00098% and 0.00195% (m/V), the secretion of inflammatory factor IL-1α by the RAW264.7 macrophages was significantly reduced, and there was a statistically significant difference compared with the NC group ($P<0.01$). It was confirmed that the polypeptide-in37 could inhibit the secretion of inflammatory factors TNF-α, IL-6 and IL-1α in the RAW264.7 macrophages, and had a soothing efficacy.

Example 6

Provided was an essence formulated with the following components by mass percentage: polypeptide-in37 0.1%, hyaluronic acid 0.08%, niacinamide 1%, p-hydroxyacetophenone 0.3%, and the balance being purified water.
The preparation method of the essence comprised: stirring the components until all the components dissolve uniformly, and then filling into a bottle to obtain a final product.

Example 7

Provided was a facial mask formulated with the following components by mass percentage: polypeptide-in37 0.2%, hyaluronic acid 0.1%, niacinamide 1%, p-hydroxyacetophenone 0.3%, and the balance being purified water.
The preparation method of the above-mentioned facial mask comprised the following steps: stirring the components until all the components dissolve evenly, immersing a mask substrate in an obtained solution, and packaging the saturated mask substrate to obtain a final product.

Example 8

Provided was a facial cream formulated with the following components by mass percentage: polypeptide-in37 0.5%, acetyl tetrapeptide 0.1%, sodium alginate 0.05%, ceramide 0.05%, glycerin 4%, sorbitol 1%, hydroxyethyl cellulose 5%, coconut oil 9%, jojoba oil 9%, vitamin E 2%, spermaceti wax 10%, para-hydroxyacetophenone 0.3%, and the balance being purified water.

The method for preparing the above-mentioned facial cream comprised the following steps:

(1) adding aqueous phase components polypeptide-in37, acetyl tetrapeptide, sodium alginate, ceramide, glycerol, sorbitol and hydroxyethyl cellulose into water, and stirring at 40 °C for 2 h;

(2) mixing oil phase components coconut oil, jojoba oil and vitamin E, and stirring at 65 °C for 2 h;

(3) under continuous stirring of the aqueous phase components, adding the oil phase components into the aqueous phase components to allow mixing and emulsification until the two phases are completely emulsified; and

(4) adding spermaceti wax and p-hydroxyacetophenone to continue mixing and emulsification under heating and stirring at 50 °C for 4 hours, and then allowing to stand for 3 h for shaping and stabilization.

The above description is only preferred embodiments of the present disclosure, but the protection scope of the present disclosure is not limited thereto. Those skilled in the art can make equivalent replacements or modifications according to the technical solution and inventive concept of the present disclosure within the technical scope disclosed by the present disclosure, which should be covered by the protection scope of the present disclosure.

## Claims

**1.** Polypeptide-in37 for use in the treatment and/or the prevention of damaged skin barrier, wherein said polypeptide-in37 has a molecular formula as follows:

$$\text{H-Ile-Leu-Pro-D-Phe-Lys-D-Phe-Pro-D-Phe-D-Phe-Pro-D-Phe-Arg-Arg-NH}_2.$$

**2.** The polypeptide-in37 for use according to claim 1, wherein said polypeptide-in37 has a concentration of 0-0.0391 mg/mL, excluding 0.

**3.** The polypeptide-in37 for use according to claim 1, wherein the polypeptide-in37 has a concentration of 0.0156-0.0313 mg/mL.

**4.** Polypeptide-in37 for use in repairing and/or soothing the skin, wherein the polypeptide-in37 has a molecular formula as follows:

$$\text{H-Ile-Leu-Pro-D-Phe-Lys-D-Phe-Pro-D-Phe-D-Phe-Pro-D-Phe-Arg-Arg-NH}_2.$$

**5.** The polypeptide-in37 for use according to claim 4, wherein the polypeptide-in37 has a concentration of 0-0.0391 mg/mL, excluding 0.

**6.** The polypeptide-in37 for use according to claim 4, wherein the polypeptide-in37 has a concentration of 0.0156-0.0313 mg/mL.

**7.** Pharmaceutical composition for use in the treatment and/or the prevention of damaged skin barrier, wherein said pharmaceutical composition comprises the polypeptide-in37 according to any one of claims 1 to 6, preferably wherein said pharmaceutical composition is a skin care product, more preferably wherein pharmaceutical composition is one selected from the group consisting of a cream formulation, an emulsion, a gel, an essence, a toner, a shampoo, a shower gel, a facial mask, and a lyophilized product.

**8.** Pharmaceutical composition for use in repairing and/or soothing the skin, wherein said pharmaceutical composition

comprises the polypeptide-in37 as defined in any one of claims 1 to 6, preferably wherein said pharmaceutical composition is a skin care product, more preferably wherein pharmaceutical composition is one selected from the group consisting of a cream formulation, an emulsion, a gel, an essence, a toner, a shampoo, a shower gel, a facial mask, and a lyophilized product.

**9.** Use of a polypeptide-in37 as defined in any one of claims 1 to 6, in the preparation of a composition with skin repairing and/or soothing efficacy.

**10.** The use according to claim 9, wherein the polypeptide-in37 has a concentration of 0-0.0391 mg/mL, excluding 0.

**11.** The use according to claim 9, wherein the polypeptide-in37 has a concentration of 0.0156-0.0313 mg/mL.

**12.** Use of a polypeptide-in37 as defined in any one of claims 1 to 6, in a cosmetic product.

**13.** Cosmetic composition comprising the polypeptide-in37 according to any one of claims 1 to 6, preferably wherein said cosmetic composition is a skin care product, more preferably wherein cosmetic composition is one selected from the group consisting of a cream formulation, an emulsion, a gel, an essence, a toner, a shampoo, a shower gel, a facial mask, and a lyophilized product.

**14.** The polypeptide-in37 for use according to any one of claims 1 to 6, or the pharmaceutical composition for use according to any one of claims 7 or 8, or the cosmetic composition according to claim 13, wherein said polypeptide-in37 is in a form including a granular form, a powdery form, or a water-soluble liquid form.

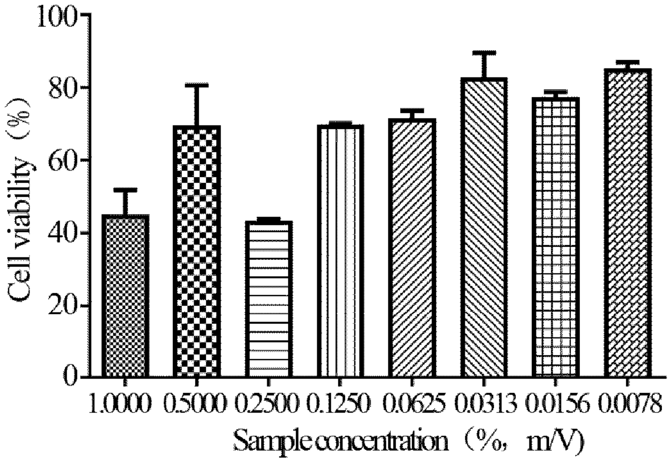
100
80
60
40
20
0
Cell viability（%）
1.0000 0.5000 0.2500 0.1250 0.0625 0.0313 0.0156 0.0078
Sample concentration（%，m/V）

FIG. 1

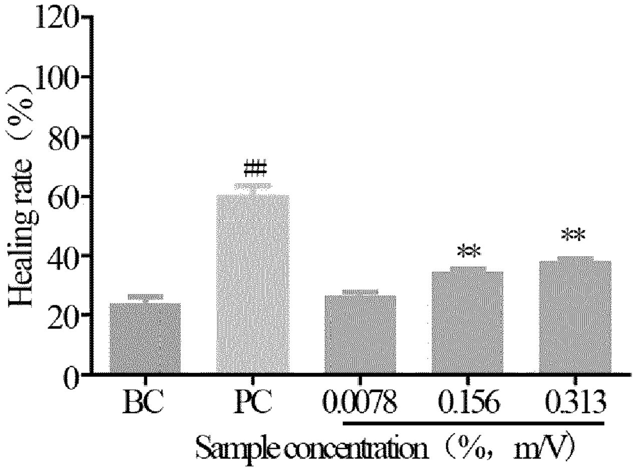
120
100
80
60
40
20
0
Healing rate（%）
##
**
**
BC
PC
0.0078
0.156
0.313
Sample concentration（%，m/V）

FIG. 2

| Group | 0 h | 24 h |
|---|---|---|
| Blank control (BC) | 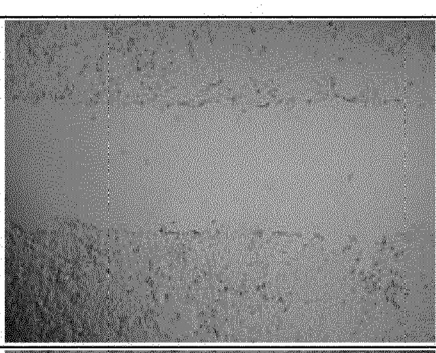 | 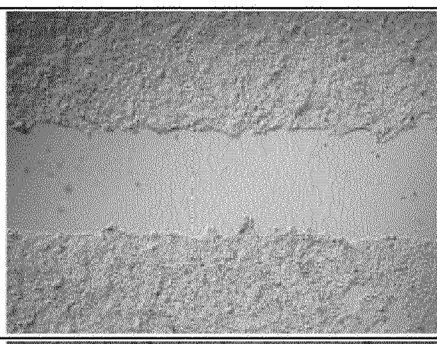 |
| Positive control (PC) | 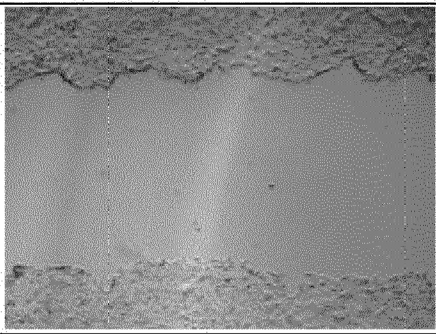 | 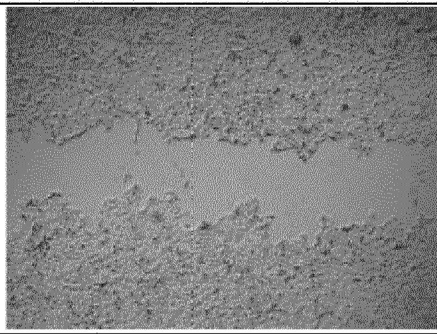 |
| Polypeptide-in37 0.0078% | 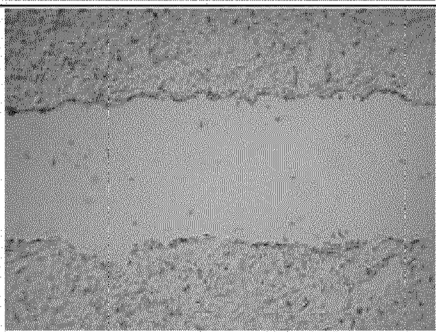 | 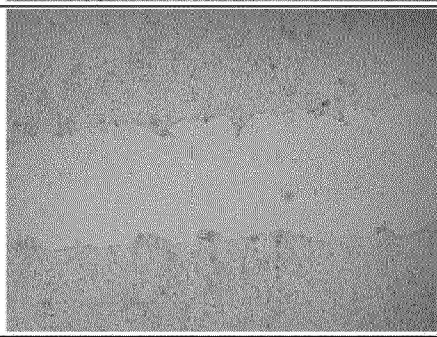 |
| Polypeptide-in37 0.0156% | 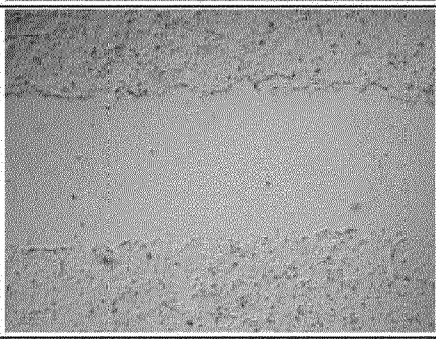 | 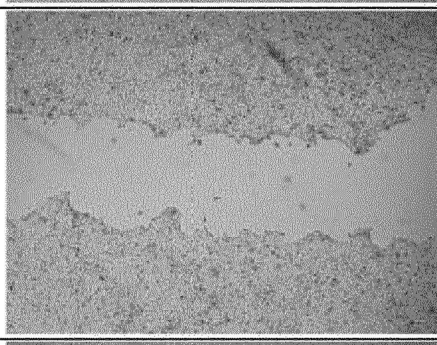 |
| Polypeptide-in37 0.0313% | 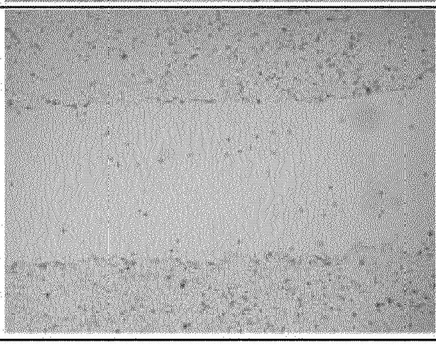 | 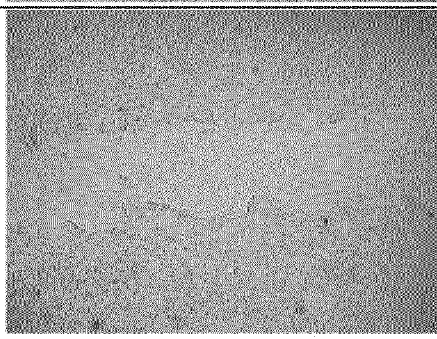 |

FIG. 3

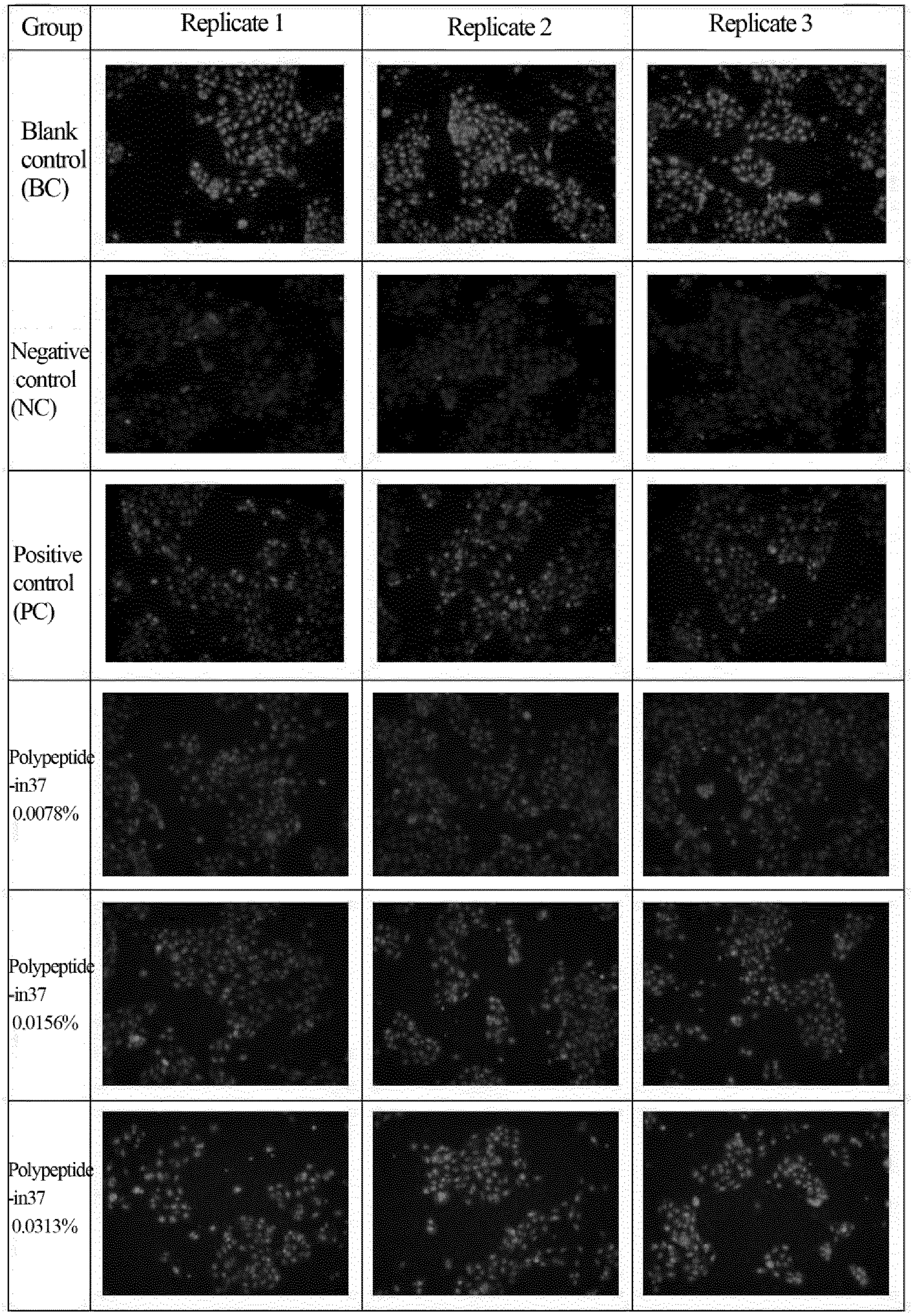
Group
Replicate 1
Replicate 2
Replicate 3
Blank control (BC)
Negative control (NC)
Positive control (PC)
Polypeptide -in37 0.0078%
Polypeptide -in37 0.0156%
Polypeptide -in37 0.0313%

FIG. 4

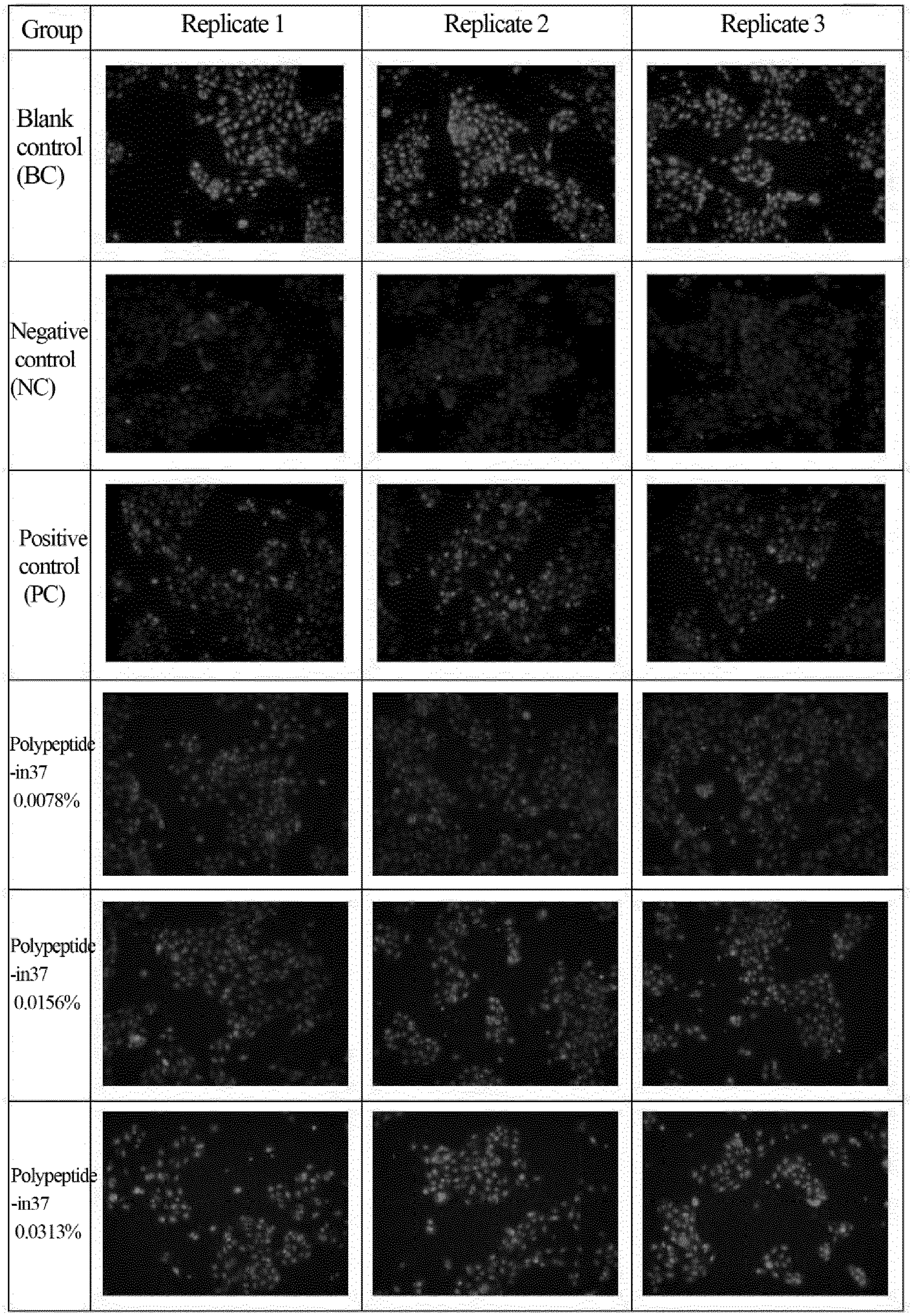
Group
Replicate 1
Replicate 2
Replicate 3
Blank control (BC)
Negative control (NC)
Positive control (PC)
Polypeptide -in37 0.0078%
Polypeptide -in37 0.0156%
Polypeptide -in37 0.0313%

FIG. 5

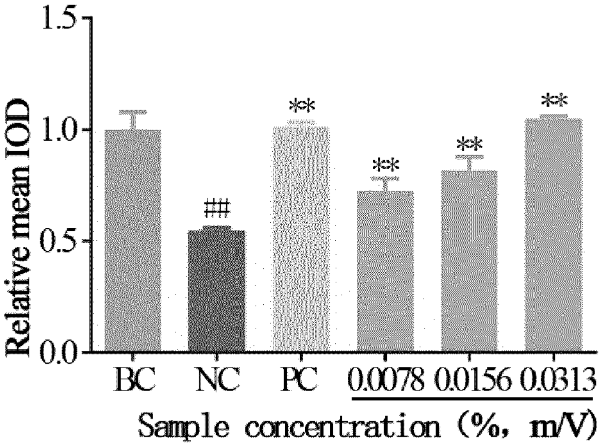
1.5
1.0
0.5
0.0
Relative mean IOD
##
**
**
**
**
BC
NC
PC
0.0078
0.0156
0.0313
Sample concentration (%, m/V)

FIG. 6

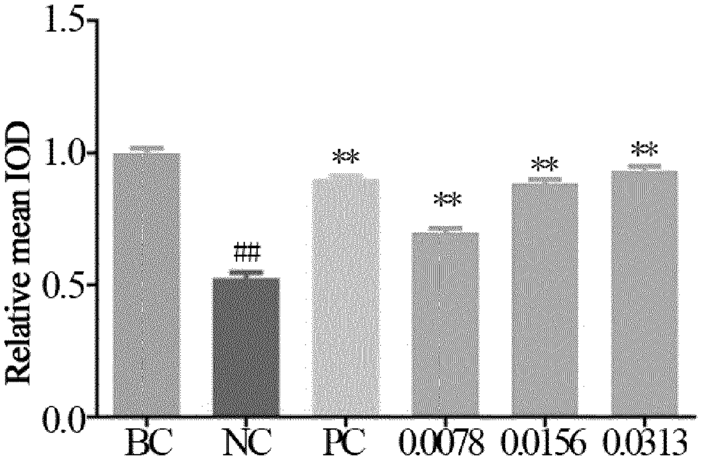
1.5
1.0
0.5
0.0
Relative mean IOD
##
**
**
**
**
BC
NC
PC
0.0078
0.0156
0.0313
Sample concentration (%, m/V)

FIG. 7

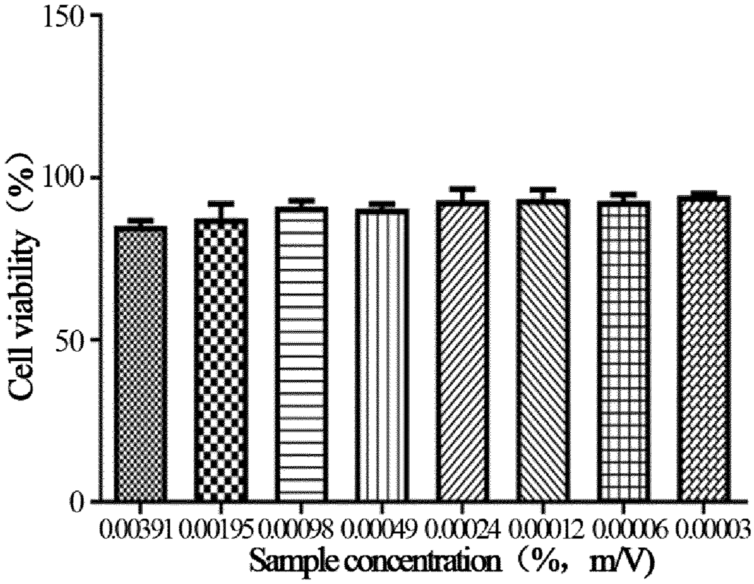
150
100
50
0
Cell viability (%)
0.00391
0.00195
0.00098
0.00049
0.00024
0.00012
0.00006
0.00003
Sample concentration (%, m/V)

FIG. 8

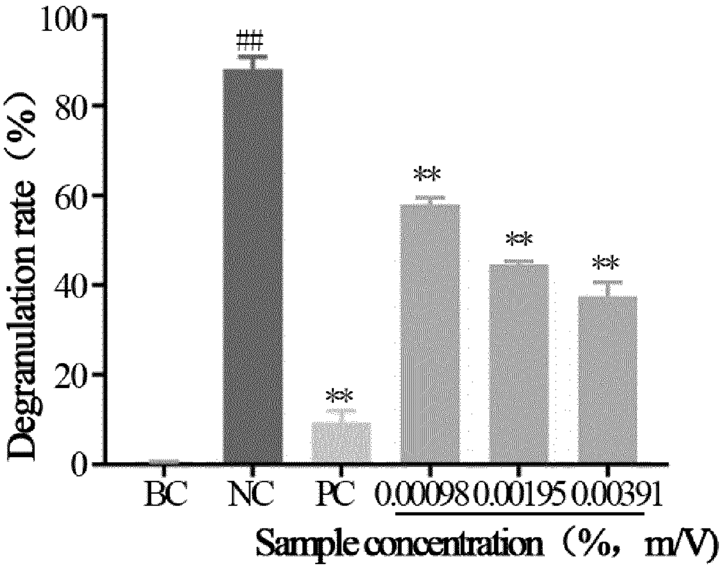
100
80
60
40
20
0
Degranulation rate (%)
##
**
**
**
**
BC
NC
PC
0.00098
0.00195
0.00391
Sample concentration (%, m/V)

FIG. 9

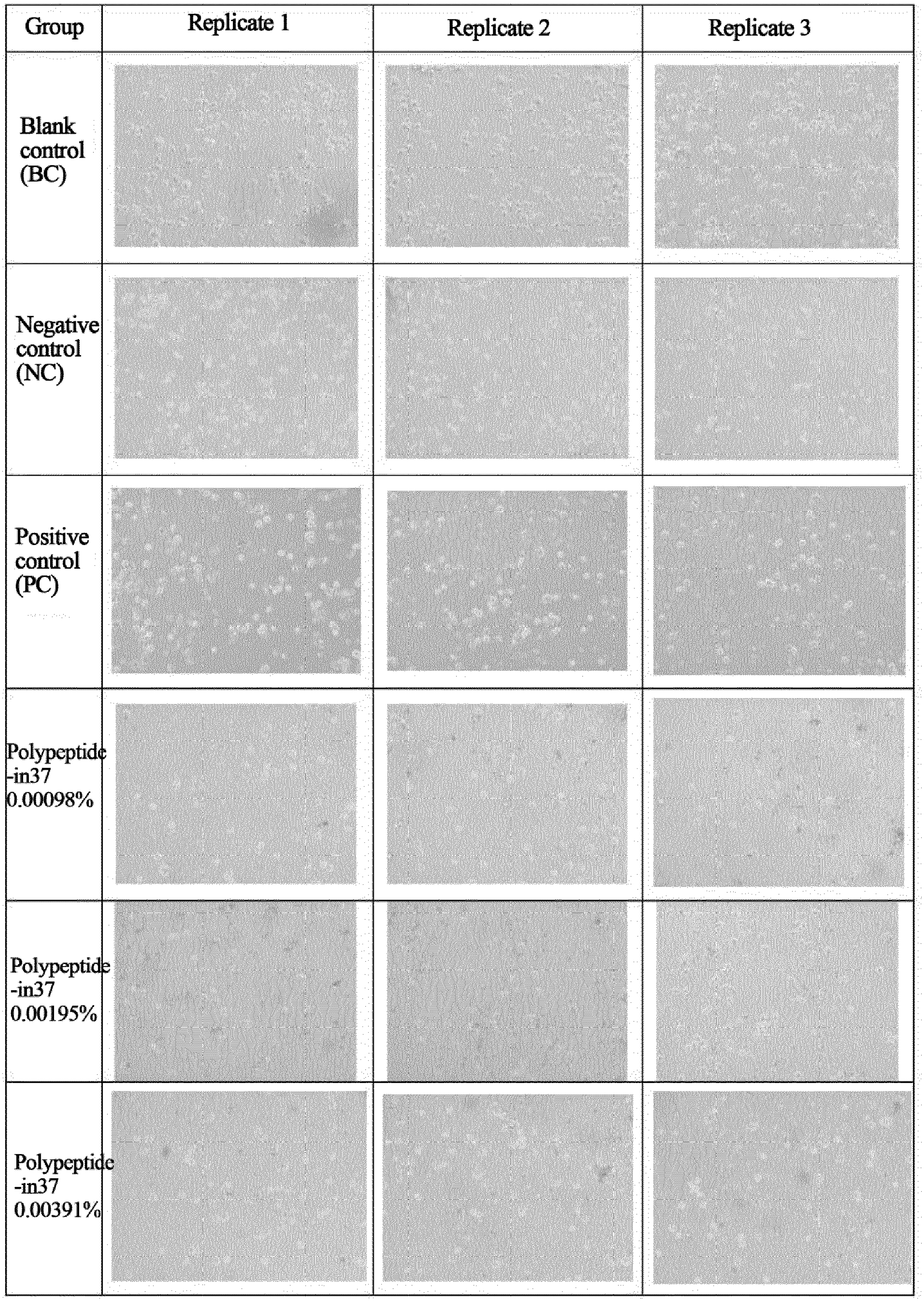
Group
Replicate 1
Replicate 2
Replicate 3
Blank control (BC)
Negative control (NC)
Positive control (PC)
Polypeptide -in37 0.00098%
Polypeptide -in37 0.00195%
Polypeptide -in37 0.00391%

FIG. 10

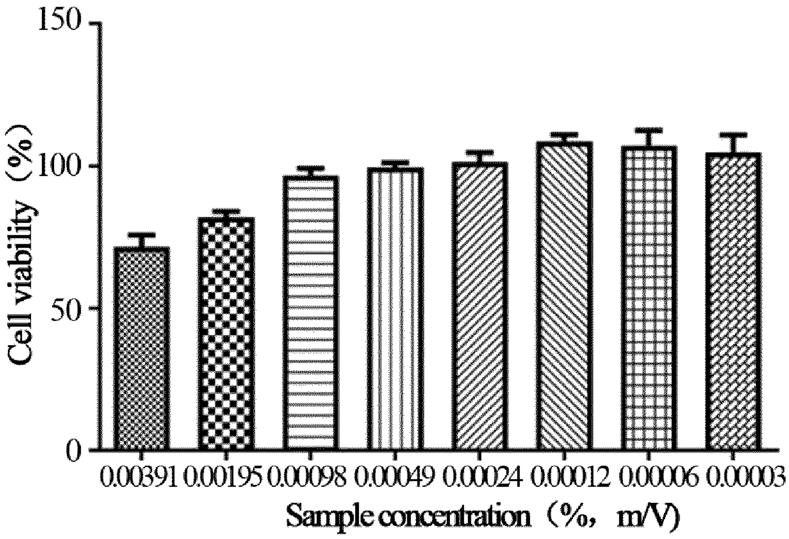
150
100
50
0
Cell viability（%）
0.00391 0.00195 0.00098 0.00049 0.00024 0.00012 0.00006 0.00003
Sample concentration（%，m/V）

FIG. 11

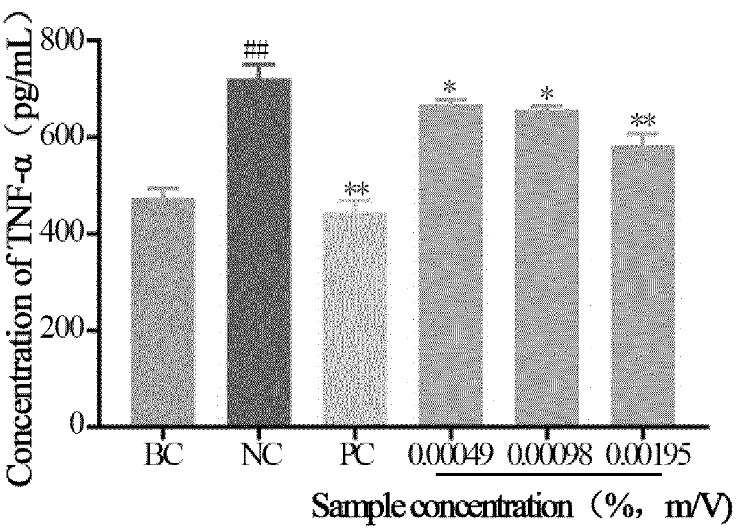
800
600
400
200
0
Concentration of TNF-α（pg/mL）
##
*
*
**
**
BC NC PC 0.00049 0.00098 0.00195
Sample concentration（%，m/V）

FIG. 12

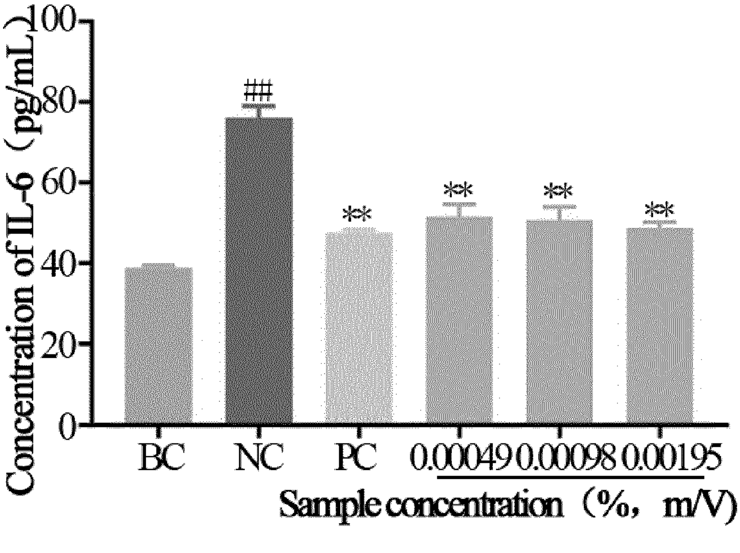
Concentration of IL-6（pg/mL）
100
80
60
40
20
0
##
**
**
**
**
BC
NC
PC
0.00049
0.00098
0.00195
Sample concentration（%，m/V）

FIG. 13

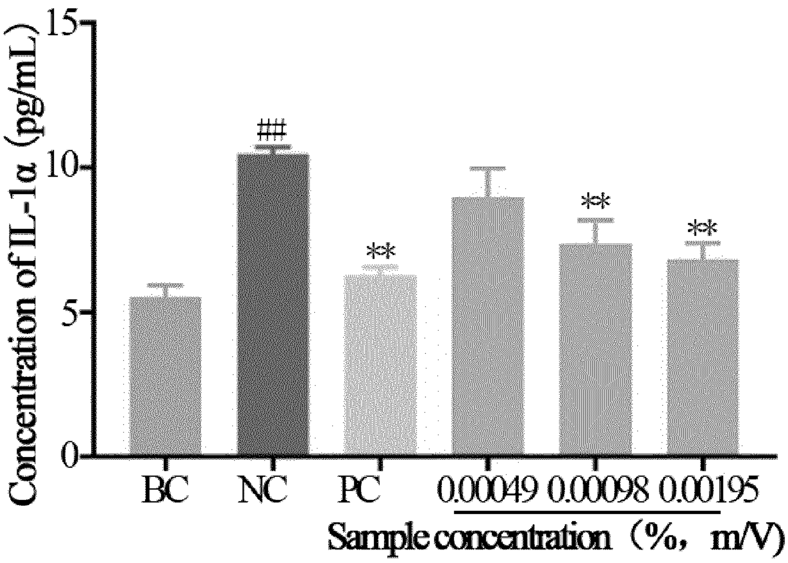
Concentration of IL-1α（pg/mL）
15
10
5
0
##
**
**
**
BC
NC
PC
0.00049
0.00098
0.00195
Sample concentration（%，m/V）

FIG. 14

Europäisches Patentamt
European Patent Office
Office européen des brevets

# EUROPEAN SEARCH REPORT

Application Number
EP 25 18 9172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | US 2020/071358 A1 (AFONINA IRINA VASIL'EVNA [RU] ET AL)<br>5 March 2020 (2020-03-05)<br>* page 2; table 1 *<br>----- | 9-14<br><br><br>1-14 | INV.<br>A61Q19/00<br>A61K8/64<br>A61K38/00<br>A61K38/04 |
| A | US 2020/046800 A1 (AFONINA IRINA VASIL'EVNA [RU] ET AL)<br>13 February 2020 (2020-02-13)<br>* page 2, paragraph 11 *<br>* page 3, paragraph 16 *<br>----- | 1-14 | |
| Y | CN 107 661 240 A (JINAN ZK COMMERCE AND TRADE CO LTD ET AL.)<br>6 February 2018 (2018-02-06)<br>* page 6, paragraph 39 *<br>* claim 1 *<br>* page 4, paragraph 6 *<br>----- | 1-14 | |
| | | | TECHNICAL FIELDS SEARCHED (IPC) |
| | | | A61Q<br>A61K |

The present search report has been drawn up for all claims

1

| Place of search | Date of completion of the search | Examiner |
|---|---|---|
| Munich | 2 December 2025 | Da Silva, Julie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 18 9172

This annex lists the patent family members relating to the patent documents cited in the above-mentioned European search report. The members are as contained in the European Patent Office EDP file on
The European Patent Office is in no way liable for these particulars which are merely given for the purpose of information.

02-12-2025

| Patent document cited in search report | Publication date | Patent family member(s) | Publication date |
|---|---|---|---|
| US 2020071358 A1 | 05-03-2020 | CN 110650970 A | 03-01-2020 |
| | | EP 3578566 A1 | 11-12-2019 |
| | | US 2020071358 A1 | 05-03-2020 |
| | | WO 2018143840 A1 | 09-08-2018 |
| US 2020046800 A1 | 13-02-2020 | CN 110114060 A | 09-08-2019 |
| | | DK 3583934 T3 | 13-09-2021 |
| | | EP 3583934 A1 | 25-12-2019 |
| | | RU 2017116079 A | 06-11-2018 |
| | | US 2020046800 A1 | 13-02-2020 |
| | | WO 2018203772 A1 | 08-11-2018 |
| CN 107661240 A | 06-02-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2018000037 W
- CN 201880003993